# EUROPEAN PATENT SPECIFICATION

(11) **EP 2 428 125 B1**
(45) Date of publication and mention of the grant of the patent: **17.05.2017**
(21) Application number: 11192130.0
(22) Date of filing: 17.09.2008
(51) Int. Cl.: A23L 33/00, A23L 33/10, A61K 38/00

(54) **Nutritional formulation with high energy content**
Ernährungsformulierung mit hohem energetischen Gehalt
Formulation nutritionnelle ayant une teneur énergétique élevée

(30) Priority: 17.09.2007 WO PCT/NL2007/000230
(43) Date of publication of application: 14.03.2012
(62) Divisional of application: 08831921.5
(73) Proprietor: N.V. Nutricia, 2712 HM Zoetermeer (NL)
(72) Inventor: Hoijer, Maarten Anne, 6814 JA Arnhem (NL); Sijben, Johannes Wilhelmus Christina, 6707 AN Wageningen (NL)
(74) Representative: Nederlandsch Octrooibureau

(56) References cited:
- WO-A-2006/091103
- WO-A-2007/046699
- WO-A-2007/073193
- WO-A1-2007/039596
- WO-A1-2007/088160
- EVANS RT AL.: "Should high-Energy infant formula be given at full strength from its first day of usage ?", THE BRITISH DIETETIC ASSOCIATION LTD, vol. 19, June 2006 (2006-06), pages 191-197, XP002727313,
- ANONYMOUS: "PENG launch the new edition of the pocket guide to clinical nutrition", PENLINES, NEWSLETTER OF THE PARENTERAL AND ENTERAL NUTRITION GROUP OF THE BRITISH DIETETIC ASSOCIATION, vol. 25, 2005, pages 1-8, XP002727314,
- ANONYMOUS: "While you're nourishing their bodies, you can help them devlop their minds, Infatrini", NUTRICAI CLINAL CARE, January 2005 (2005-01), XP002727315,
- BOERSMA ET AL.: "Catch-up growth", ENDOCRINE REVIEWS, vol. 18, no. 5, 1997, pages 646-661, XP002727316,
- anonymous: "Faltering growth", The children's society and community practionners and health visitors association , 2010, XP002727317, Retrieved from the Internet: URL:http://http://www.gp-training.net/trai ning/tutorials/clinical/paediatrics/pgrowt h2.htm [retrieved on 2014-07-16]

## Description

### Field of the invention

Infants suffering from disease commonly show faltering growth patterns that can be corrected with additional high energy nutrition. The present invention relates to compositions that promote catch-up growth in such infants without the negative side effects on the intestinal barrier function commonly associated with energy dense compositions.

### Background of the invention

In the first year of life, children (infants) are particularly sensitive to energy and nutrient restriction because of high basal anabolic requirements. Energy and nutrient requirements per unit of body weight are greatest during the first 4 to 6 months of life, with a gradual decrease in requirements until the adolescent growth spurt.

There is now considerable evidence to show that the primary driving force for growth in infancy is adequate nutrition. In later childhood and adolescence humoral factors, such as growth hormone, thyroxin and cortisol, assume a much more important role but adequate nutrition is still required.

Infants requiring nutritional support present unique challenges, not only because of their high nutrient requirements for growth, development and organ maturation, but also because of their small body reserves. Infants have fewer body reserves of all nutrients than adults, particularly energy, and these resources can be depleted rapidly during acute and chronic disease. Tissue wasting to meet energy demands proceeds much more rapidly in infants than in older children and adults, which makes them particularly susceptible to the effects of starvation. It has been estimated that an adult has sufficient body reserves for approximately 70 days, compared with 4 days in a preterm baby and 31 days in a full-term baby.

During acute and chronic disease infants have specific nutritional requirements and often suffer from faltering growth. Therefore these infants will need extra nutrition to compensate for the lack of growth which should have taken place during disease. Normally this is done by giving extra energy. Since infants don't drink large volumes the energy is provided in concentrated drink formula, typically comprising at least 0.9 kcal/ml.

WO 2007/039596 discloses such a composition comprising probiotic bacteria, prebiotic fibers and LC-PUFA as active ingredients for promoting catch-up growth in young mammals which have been subject to physical or mental stress.

WO 2006/091103 discloses compostions comprising probiotic bacteria and prebiotic fibers as active ingredients for the treatment and/or prevention of gastrointestinal disorders, imune disorders and/or endocrine disorders.

WO 2007/073193 discloses compostions to be adminstered to infants comprising a blend of lipds and uridine as active ingredients for preventing obesity later in life. WO 2007/046699 discloses compositions comprising LC-PUFA and/or nucleotides for developing intestinal flora in infants delivered via ceaserean section. Evans et al., "Should high-energy infant formula be given at full strength from its first day of usage?"; THE BRITISH DIETETIC ASSOCIATION LTD, vol. 19, June 2006, pages 191-197, discloses the use of the formulation Infatrini® for infants with faltering growth. An object of the present invention is to provide a formulation suitable to promote optimal catch-up growth in infants, preferably in the age group of 0 -18 months, which suffer from faltering growth due to illness, while minimising gastrointestinal symptoms commonly associated with energy dense compositions.

### Summary of the invention

The inventors recognized the need for improving current practice of feeding infants for treating or preventing faltering growth during or after a period of disease. In particular it was recognized that energy dense formulations result in poor intestinal functioning, particularly due to a decreased uptake of nutrients; decreased barrier function and/or increased growth of undesirable bacteria.

Normally, infant nutrition has an energy density of about 0.7 kcal/ml. The present group of patients needs an increased energy density. The present inventors have recognized that the increased energy density can lead to an increased growth of undesirable bacteria in the intestinal tract because not all nutrients (e.g. proteins) are readily absorbed. The present inventors found that the side effects can be minimized by including a synergistically acting mixture of nucleotides, LC-PUFA's and non-digestible oligosaccharides, i.c. indigestible carbohydrates in the present energy dense composition. Also the present inventors recognized that it is not beneficial to administer live bacteria to sick infants.

Accordingly, the present invention provides a nutritional formulation for promoting catch-up growth in infants comprising a source of lipids containing long-chain polyunsaturated fatty acid (LC-PUFA) in an amount of at least 0.1 wt% of the fatty acids in the composition, indigestible carbohydrates (prebiotic fibers) in an amount of at least 0.1 wt% based on dry weight of the composition and nucleotides in an amount of at least 1.10⁻⁴ wt% based on dry weight of the composition.

A particular advantage of the present invention is that it provides a composition capable of promoting catch-up growth, in particular in sick infants, by improving the intake of protein and at the same time improving the uptake of nutritional ingredients by improving the absorption in the gut. This ultimately leads to minimising gastrointestinal symptoms, also by the synergistic action in support of the (systemic) immune system of the infant by the presence of the nucleotides, supporting developmentally appropriate feeding behaviour, and enhancing the quality of life.

### Detailed description of the invention

In one embodiment the present invention concerns the use of a composition comprising protein, fat, digestible and indigestible carbohydrates and nucleotides for the manufacture of a high energy content composition for the treatment and/or prevention of faltering growth in infants, wherein the composition has
a. an energy density of at least 0.9 kcal/ml and preferably below 2.0 kcal/ml; and
b. a protein component providing at least 9.5 % of the total calories; and
c. at least 0.1 wt% long chain polyunsaturated fatty acids based on total fatty acids; and
d. at least 0.1 wt% indigestible carbohydrate based on the dry weight of the composition; and
e. at least 1.10⁻⁴ wt% nucleotides based on the dry weight of the composition.

In one embodiment the present invention concerns a method for the treatment and/or prevention of faltering growth in infants, said method comprising administering a high energy content composition as defined herein to said infant.

In one embodiment the present invention concerns a composition as defined herein for use in the treatment and/or prevention of faltering growth in infants. In one embodiment the use is for promoting catch-up growth in a sick infant.

In one embodiment the composition has an energy density of at least 0.9 kcal/ml and below 1.8 kcal/ml, preferably of at least 0.9 kcal/ml and below 1.5 kcal/ml. In one embodiment the composition has an energy density of at least 0.9 kcal/ml and below 1.2 kcal/ml.

### Indigestible carbohydrate

The indigestible carbohydrate is preferably selected from the group consisting of fructo-oligosaccharides, galacto-oligosaccharides, sialo- oligosaccharides, xylo-oligosaccharides, inulin, arabic gum, guar gum, resistant starch, milk- derived oligosaccharides and mixtures thereof. Preferably the indigestible carbohydrate is selected from the group consisting of fructo-oligosaccharides, inulin, gum Arabic and galacto-oligosaccharides. Preferably the present composition comprises galactooligosaccharides. These carbohydrates are particularly effective in stimulating the growth of Bifidocateria and Lactobacilli. Preferably the present composition comprises (i) galacto-oligosaccharide and (ii) fructo-oligosaccharide and/or inulin. Preferably the weight ratio galactooligosaccharides : (fructooligosaccharides and/or inulin) is between: 25:1 and 1:25. Suitable galacto-oligosaccharide is commercially available from Borculo Domo Ingredients under the trade mark Vivinal GOS 10. A suitable fructo-oligosaccharide is commercially available from Orafti S. Preferably, the indigestible carbohydrate(s) is/are present in a total amount of of at least 0.1 wt% based on the dry weight of the composition.

The present invention preferably comprises the administration of a serving comprising between 0.05 and 25 grams indigestible carbohydrate, preferably between 0.1 and 5 grams. The present invention preferably comprises the administration of a serving comprising between 0.05 and 25 grams galacto-oligosaccharides, preferably between 0.1 and 5 gram galacto-oligosaccharides. The present invention preferably comprises the administration of 0.05 to 25 grams indigestible carbohydrate per day, preferably between 0.1 and 5 grams per day. The present invention preferably comprises the administration between 0.05 and 25 grams galacto-oligosaccharides per day, preferably between 0.1 and 5 gram galacto-oligosaccharides per day.

Preferably the present indigestible carbohydrates have a degree of polymerisation (DP) of at least 2 monose units and are not or only partially digested in the intestine by the action of acids or digestive enzymes present in the human upper digestive tract (small intestine and stomach), but which are fermentable by the human intestinal flora. The term monose units refers to units having a closed ring structure, preferably hexose, e.g. the pyranose or furanose forms. The average degree of polymerisation of the oligosaccharide is typically below 60 monose units, preferably below 40, even more preferably below 20.

According to a further embodiment at least one of the indigestible carbohydrate of the present composition is selected from the group consisting of fructans, fructooligosaccharides, indigestible dextrins, galactooligosaccharides (including transgalactooligosaccharides), xylooligosaccharides, arabinooligosaccharides, glucooligosaccharides, mannooligosaccharides, fucooligosaccharides, acidic oligosaccharides (e.g. uronic acid oligosaccharides such as pectin hydrolysate) and mixtures thereof. Preferably the present composition comprises at least one, preferably at least two, of the indigestible carbohydrates selected from the group consisting of fructooligosaccharides or inulin, galactooligosaccharides and pectin and/or pectin hydrolysate.

According to a further preferred embodiment the present composition comprises at least one of the indigestible carbohydrates selected from the group consisting of fructo-oligosaccharides, inulin, gum Arabic and galacto-oligosaccharides and a pectin hydrolysate. Preferably at least 70% of the pectin hydrolysate has a degree of polymerization between 2 and 50. The average molecular weight of the pectin hydrolysate is preferably between 1 kD and 20 kD, preferably between 4 and 10 kD. The average molecular weight can be determined by the method as described in US 5,472,952.

When in ready-to-feed liquid form, the present composition preferably comprises 0.1 to 100 grams indigestible carbohydrate per liter, more preferably between 0.5 and 50 grams per liter even more preferably between 1 and 25 grams per liter. A too high content of indigestible carbohydrate may cause discomfort due to excessive fermentation, while a very low content may result in an insufficient mucus layer.

In case at least two different indigestible carbohydrate are present, the weight ratio is preferably between 1 and 10, more preferably between 1 and 5. These weight ratios stimulate mucin production of different types at different sites in the intestine optimally.

The indigestible carbohydrate is preferably included in the present composition in an amount exceeding 0.1 wt.%, preferably exceeding 0.2 wt.%, more preferably exceeding 0.5 wt.% and even more preferably exceeding 1 wt.% based on the total dry weight of the composition. The present composition preferably has an indigestible carbohydrate content below 20-wt.%, more preferably below 10-wt.% even more preferably below 5-wt.%.

### Long chain polyunsaturated fatty acids

The present composition preferably comprises long chain polyunsaturated fatty acids (LC-PUFA). LC-PUFA are fatty acids wherein the acyl chain has a length of 20 to 24 carbon atoms (preferably 20 or 22 carbon atoms) and wherein the acyl chain comprises at least two unsaturated bonds between said carbon atoms in the acyl chain. More preferably the present composition comprises at least one LC-PUFA selected from the group consisting of eicosapentaenoic acid (EPA, 20:5 n3), docosahexaenoic acid (DHA, 22:6 n3), arachidonic acid (ARA, 20:4 n6) and docosapentaenoic acid (DPA, 22:5 n3). EPA, DHA and ARA were found to effectively reduce intestinal tight junction permeability. Reduced tight junction permeability reduces the occurrence of infection and/or reduces passage of bacterial (endo- or exo)toxins. Hence incorporation of LC-PUFA, preferably EPA, DHA, DPA and/or ARA, in the present composition improves intestinal barrier integrity, which is of utmost important in ill infants since these babies have a less developed intestinal flora and hence a slower maturing intestinal barrier. LC-PUFA further have antiinflammatory effects and promote the adhesion of lactic acid producing bacteria to mucosal surfaces, thereby stimulating the development of a healthy flora.

Since a low concentration of ARA, DHA, DPA and/or EPA is already effective in reducing tight junction permeability, the content of LC-PUFA in the present composition, which is preferably a nutritional composition, preferably does not exceed 5 wt.% of the total fat content, preferably does not exceed 2.5 wt.%, even more preferably does not exceed 1 wt.%. Preferably the present composition comprises at least 0.1 wt.%, preferably at least 0.25 wt.%, more preferably at least 0.5 wt.%, even more preferably at least 0.75 wt.% LC-PUFA of the total fat content. For the same reason, the EPA content preferably does not exceed 1 wt.% of the total fat, more preferably does not exceed 0.5 wt.%, but is preferably at least 0.02 wt.%, more preferably at least 0.05 wt.% of the total fat. The DHA content preferably does not exceed 1 wt.%, more preferably does not exceed 0.5 wt.%, but is at least 0.1 wt.% of the total fat. As ARA was found to be particularly effective in reducing tight junction permeability, the present composition comprises relatively high amounts, preferably at least 0.1 wt.%, even more preferably at least 0.25 wt.%, most preferably at least 0.35 wt.% ARA of the total fat. The ARA content preferably does not exceed 1 wt.% of the total fat. When the present enteral composition comprises ARA, EPA and DHA are advantageously added to balance the action of ARA, e.g. reduce the potential pro-inflammatory action of ARA metabolites. Excess metabolites from ARA may cause inflammation. Hence, the present composition preferably comprises ARA, EPA and DHA, wherein the weight ratio ARA/DHA preferably is above 0.5, preferably above 0.7, even more preferably above 1. The ratio ARA/DHA is preferably below 10,. The weight ratio ARA/EPA is preferably between 1 and 100, more preferably between 5 and 20.

The n6:n3 ratio is preferably between 1:2 to 8:1, more preferably between 4:1 to 8:1. The source of the LC-PUFA may be, for example, egg lipids, fungal oil, low EPA fish oil or algal oil.

Importantly the ratio linoleic acid (C18:2 n6) to alpha linolenic acid (C18:3 n-3) is preferably between 4 and 6, more preferably between 4.5 and 5.5. This ratio will provide the optimal fat mixture for the treatment of intestinal barrier function, and thus for the induction of a healthy catch-up growth.

### Nucleotides

Addition of nucleotides and/or nucleosides to the present composition further improves gut mucosal barrier function, particularly as it inhibits and/or or reduces the incidence of bacterial translocation and decreases intestinal injury. Hence, the present composition also comprises at least 1.10⁻⁴ wt%, preferably between 1 and 500 mg nucleosides and/or nucleotides total weight of the dry formula, even more preferably between 5 and 100 mg. Advantageously the present composition includes nucleotides and/or nucleosides since in combination with the LC-PUFA and indigestible carbohydrates these components unexpectedly improve gut barrier function, immune system and/or intestinal flora in what is believed to be a synergistic effect.

More preferably the composition comprises nucleotide. The nucleotide is preferably in the monophosphate, diphosphate or triphosphate form, more preferably a nucleotide monophosphate. The nucleotide preferably is a ribonucleotide or a deoxyribonucleotide, more preferably a ribonucleotide. The nucleotides can be monomeric, dimeric or polymeric (including RNA and DNA). The nucleotides preferably are present as a free acid or in the form of a salt, more preferably monosodium salt. Incorporation of nucleotide in the present composition improves intestinal barrier integrity and/or maturation, which is of utmost importance for ill infants since these infants have a less developed intestinal flora and hence a slower maturing intestinal barrier. Preferably, the composition comprises one or more selected form the group consisting of cytidine 5'-monophospate (CMP), uridine 5'-monophospate (UMP), adenosine 5'-monophospate (AMP), guanosine 5'-monophospate (GMP), and inosine 5'-monophospate (IMP), more preferably at least 3 selected form the group consisting of CMP, UMP, AMP, GMP and IMP. An increased diversity of nucleotides can further improve barrier integrity.

Preferably the composition comprises 5 to 100 mg, more preferably 5 to 50 mg, most preferably 10 to 25 mg nucleotides per 100 gram dry weight of the present composition. The nucleotides further stimulate the immune system thereby enhancing protection against a high load of intestinal pathogens such as *E. coli.*

Preferably the present composition comprises 1 to 20 mg, more preferably 3 to 12.5 mg CMP per 100 g dry weight of the composition. Preferably the composition comprises 1 to 20 mg, more preferably 2 to 9 mg UMP per 100 g dry weight of the composition. Preferably the present composition comprises 0.5 to 15 mg, more preferably 1.5 to 8 mg AMP per 100 g dry weight of the present composition. Preferably the composition comprises 0.2 to 10 mg, more preferably 0.6 to 3 mg GMP per 100 g dry weight of the composition. Preferably the present composition comprises 0.5 to 10 mg, more preferably 1.3 to 5 mg IMP per 100 g dry weight of the composition.

Accordingly a preferred composition according to the invention comprises nucleotides, protein, fatty acids, digestible and indigestible carbohydrates and has
a. an energy density of 0.95-1.15 kcal/ml; and
b. a protein component providing at least 9.5 % of the total calories, preferably between 10 and 12 en%;
c. between 0.1 - 0.5 wt% long chain polyunsaturated fatty acids based on total fatty acids in the composition;
d. between 0.2 - 0.4 wt% indigestible carbohydrate based on the dry weight of the composition; and
e. at least 3 nucleotides selected form the group consisting of cytidine 5'-monophospate (CMP), uridine 5'-monophospate (UMP), adenosine 5'-monophospate (AMP), guanosine 5'-monophospate (GMP), and inosine 5'-monophospate (IMP) present in an amount of at least 1.10⁻⁴ wt% based on the dry weight of the composition, preferably in an amount of 2.10⁻⁴ and 4.10⁻⁴ wt% based on the dry weight of the composition.

### Macronutrients

The present composition preferably provides nutrition to the infant, and comprises a lipid component, a protein component and a carbohydrate component. The lipid component preferably provides 5 to 50% of the total calories, the protein component preferably provides 9.5 to 50% of the total calories, and the carbohydrate component preferably provides 15 to 90% of the total calories. The present composition is preferably used as an infant formula, wherein the lipid component provides 35 to 50% of the total calories, the protein component provides 10 to 12.5% of the total calories, and the carbohydrate component provides 40 to 55% of the total calories. For calculation of the % of total calories for the protein component, the total of energy provided by the proteins, peptides and amino acids needs to be taken.

The protein component used in the present composition preferably comprises at least one selected from the group consisting of non-human animal proteins (such as milk proteins, meat proteins and egg proteins), vegetable proteins (such as soy protein, wheat protein, rice protein, potato protein and pea protein), free amino acids and mixtures thereof. Cow's milk derived nitrogen source, particularly cow milk protein proteins such as casein and whey proteins are particularly preferred. Preferably the protein component comprises intact proteins, more preferably intact bovine whey proteins and/or intact bovine casein proteins.

Preferably the present composition is not mammalian milk, preferably not human milk. Preferably the present composition does not contain human milk protein.

Osmolarity is an important factor determining the gastro-intestinal tolerance. In order to improve gastro-intestinal tolerance the osmolarity of the composition preferably does not exceed 350 mOsmol/Liter, more preferably less than 300 mOsmol/Liter. If the composition is used as a tube feed, the osmolarity preferably is less than 300 mOsmol/Liter, even more preferably less than 250 mOsmol/Liter since tube fed infants are more sensitive to feedings with a higher osmolarity.

Sick infants often suffer from a decreased intestinal barrier function. In order to prevent unwanted gastro-intestinal infections the composition according to the invention preferably does not comprise live probiotic bacteria.

### Application

The present composition aims to provide nutrition to a sick infant and/or the treatment and/or prevention of faltering growth in infants. The present composition is particularly suitable for administration to an infant suffering from cystic fibrosis, heart disorder, trauma, cancer and/or palsy or has been or will be subjected to surgery. Preferably the present composition is administered via a gastrointestinal tube. The present composition is preferably administered in a daily dosage of between 100 ml and 1000 ml. Preferably the composition is administered to an infant of the age between 0 and 18 months. In one embodiment the present composition does not comprise live bacteria.

Administration of the present composition comprising LC-PUFA, indigestible oligosaccharides and nucleotide results in a decreased translocation of (nosocomial) pathogenic micro-organisms, such as *Eschericia coli,* other Gram-negative species belonging to the genera *Aeromonas, Klebsiella, Enterobacter, Pseudomonas, Proteus,* and *Acinetobacter* and Gram-positive species such as *Enterococcus, Staphylococcus, Streptococcus, Bacillus,* and *Clostridium,* viruses and/or fungi, preferably *Escherichia coli* (*E. coli*). Hence, the present composition is preferably used in a method for treatment and/or prevention of infection and/or diarrhea in ill infants or infant suffering from faltering growth, said method comprising administering the present composition to an infant delivered by caesarean section. In a preferred embodiment the present composition is used for the treatment and/or prevention of infection caused by *Eschericia coli, Aeromonas, Klebsiella, Enterobacter and*/*or Pseudomonas,* more preferably *E. coli.*

The gastro-intestinal tolerance is improved by using the compositions according to the invention. Therefore the composition according to the invention is preferably used for improving the gastro-intestinal tolerance to enteral fed formula in ill infants.

### Examples

The following non-limiting examples further illustrate the invention.

### Example 1: Nutritional compositions - Infatrini

Composition with an energy density of 1kcal/ml suitable for inducing catch-up growth in patients between 0 and 18 months

| | | g/100ml |
|---|---|---|
| Protein | 10.4 en% | 2.6 |
| Carbohydrate | 41.1 en% | 10.3 |
| Fat | 48.6 en% | 5.4 |
| Fibre | | 0.8 |
| Nucleotides | | 2.8 .10⁻³ (CMP, UMP, AMP, GMP, IMP) |
| Vit/mineral according to EC directive FSMP 1999/21 | | |

| COMPONENT UNIT | Per 100 ml | Per 100 kcal |
|---|---|---|
| ***Protein (equivalent), total* g** | 2.60 | 2.61 |
| - Nitrogen (Protein) g | 0.41 | 0.41 |
| - Animal protein g | 2.60 | 2.61 |
| • Whey protein g | 1.56 | 1.57 |
| • Casein g | 1.04 | 1.04 |
| ***Carbohydrate* g** | 10.3 | 10.3 |
| - Sugars g | 5.69 | 5.71 |
| • Glucose g | 0.28 | 0.28 |
| • Lactose g | 5.16 | 5.18 |
| • Maltose g | 0.24 | 0.24 |
| - Polysaccharides g | 4.42 | 4.43 |
| ***Fat* g** | 5.36 | 5.38 |
| - Vegetable g | 5.24 | 5.26 |
| - Animal g | 0.12 | 0.12 |
| • of which milk g | 0.08 | 0.08 |
| - Saturates g | 2.05 | 2.05 |
| • of which MCT g | | |
| - Monounsaturates g | 2.33 | 2.34 |
| - Polyunsaturates g | 0.99 | 0.99 |
| *Fibre, dietary* g | 0.800 | 0.803 |
| - Soluble g | 0.80 | 0.80 |
| *Moisture*/*water* g | 84.9 | 85.2 |
| | | |
| *Nucleotides* ** mg | 2.80 | 2.81 |
| - Cytidine-5'-monophosphate | 0.93 | 0.94 |
| - Uridine-5'-monophosphate | 0.47 | 0.47 |
| - Adenosine-5'-monophosphate | 0.78 | 0.78 |
| - Guanosine-5'-monophosphate | 0.23 | 0.23 |
| - Inosine-5'-monophosphate | 0.39 | 0.39 |

### Osmolarity: 290 mOsmol/Liter

### Example 2. Reduced intestinal tight junction permeability with fatty acid mixture comprising of EPA, DHA and ARA.

Monolayers (MC) of intestinal epithelial cell lines T84 (ATCC, USA) were cultured on transwell filters (Corning, Costar BV, The Netherlands) allowing both mucosal and serosal sampling and stimulation of human intestinal epithelial cells. Two weeks post confluency the monolayers were incubated in the presence of IL-4 (2 ng/ml, serosal compartment, Sigma, USA) with or without polyunsaturated fatty acids ARA, DHA, GLA, EPA, or control palmitic acid (10 µM or 100 µM, mucosal compartment, Sigma, St. Louis, USA). Cells were pre-incubated with ARA, DHA, EPA, or palmitic acid for 48 hr prior to the IL-4 incubation. The co-incubation of PUFA's and palmitic acid with IL-4 was continued for another 48 hr; while culture medium and additives were changed every 24 hr. The epithelial barrier function was determined by measuring the transepithelial resistance (TER) and permeability.

Results of the effect of ARA, DHA, EPA and palmitic acid (100 µM) on IL-4 mediated barrier disruption are given in Table 1. Table 1 shows that the LC-PUFA's ARA, DHA and EPA inhibit the increased flux caused by IL-4. In contrast palmitic acid had a detrimental effect and decreased barrier disruption compared to control. These results are indicative for the advantageous use of ARA, DHA, and EPA in clinical and infant nutrition formulations to prevent or reduce IL-4 mediated barrier disruption. These result further support the synergistic effects of the present combination of fatty acids and indigestible oligosaccharides.

**Table 1**

| Ingredient (LC-PUFA) | IL-4 Flux | IL-4 TER |
|---|---|---|
| Control | 582 | 374 |
| Palmitic acid | 777 | 321 |
| DHA | 271 | 547 |
| ARA | 218 | 636 |
| EPA | 228 | 539 |

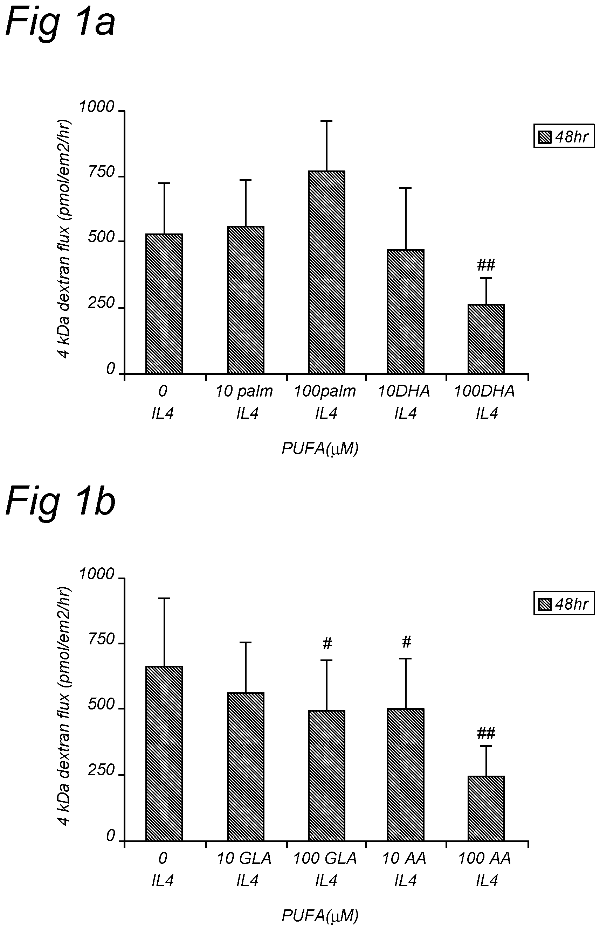

Figure 1 gives the time and dose (10µM and 100µM) dependent protective effects of various FA's (palmitic acid, DHA, GLA, and AA) on IL-4 mediated barrier destruction (Flux). Figure 1 shows that ARA, DHA and GLA protect against IL-4 mediated barrier disruption as reflected by decreased 4kD dextran flux. These results are indicative for the advantageous use of ARA, DHA and GLA in clinical and infant nutrition formulations to prevent or reduce IL-4 mediated barrier disruption, e.g. as occurs in food or cows milk allergy. These result further support the synergistic effects of the present combination of fatty acids and indigestible carbohydrates.

## Claims

1. A high energy content composition comprising protein, fat, digestible and indigestible carbohydrates and nucleotides, wherein the composition has
a. an energy density of at least 0.9 kcal/ml and preferably below 2.0 kcal/ml; and
b. a protein component providing at least 9.5 % of the total calories; and
c. at least 0.1 wt% long chain polyunsaturated fatty acids (LC-PUFAs) based on total fatty acids, comprising at least one LC-PUFA selected from the group consisting of eicosapentaenoic acid (EPA), docosahexaenoic acid (DHA), arachidonic acid (ARA) and docosapentaenoic acid (DPA); and
d. at least 0.1 wt% indigestible carbohydrate based on the dry weight of the composition; and
e. at least 1.10⁻⁴ wt% nucleotides based on the dry weight of the composition
for use in the treatment and/or prevention of faltering growth in infants.

2. The composition for use according to claim 1 wherein the composition is administered as a tube feed.

3. The composition for use according to claim 1 or 2, wherein the indigestible carbohydrate is selected from the group consisting of fructo-oligosaccharides, galacto-oligosaccharides, sialo-oligosaccharides, xylo-oligosaccharides, inulin, arabic gum, guar gum, resistant starch, milk-derived oligosaccharides, pectin and mixtures thereof.

4. The composition for use according to any one of the preceding claims wherein the composition comprises at least 3 nucleotides selected form the group consisting of cytidine 5'-monophospate (CMP), uridine 5'-monophospate (UMP), adenosine 5'-monophospate (AMP), guanosine 5'-monophospate (GMP), and inosine 5'-monophospate (IMP).

5. The composition for use according to any one of the preceding claims wherein the composition is for promoting catch-up growth in a sick infant.

6. The composition for use according to any one of the preceding claims wherein the composition does not comprise live bacteria.

## Patentansprüche

1. Zusammensetzung mit hohem Energiegehalt, umfassend Protein, Fett, verdauliche und unverdauliche Kohlenhydrate und Nukleotide, wobei die Zusammensetzung
a. eine Engergiedichte von mindestens 0,9 kcal/ml und bevorzugt unter 2,0 kcal/ml; und
b. einen Proteinbestandteil, der mindestens 9,5% der Gesamtkalorien bereitstellt; und
c. mindestens 0,1 Gew.-% langkettige polyungesättigte Fettsäuren (LC-PUFAs) basierend auf den Gesamtfettsäuren, umfassend mindestens eine LC-PUFA ausgewählt aus der Gruppe bestehend aus Eicosapentaensäure (EPA), Docosahexaensäure (DHA), Arachidonsäure (ARA) und Docosapentaensäure (DPA); und
d. mindestens 0,1 Gew.-% unverdauliches Kohlenhydrat basierend auf dem Trockengewicht der Zusammensetzung; und
e. mindestens 1.10⁻⁴ Gew.-% Nukleotide basierend auf dem Trockengewicht der Zusammensetzung
aufweist, zur Verwendung in der Behandlung und/oder Vorbeugung von Wachstumsverzögerung in Kindern.

2. Zusammensetzung zur Verwendung nach Anspruch 1, wobei die Zusammensetzung als eine Magensondenzufuhr verabreicht wird.

3. Zusammensetzung zur Verwendung nach Anspruch 1 oder 2, wobei das unverdauliche Kohlenhydrat ausgewählt ist aus der Gruppe bestehend aus Fructooligosacchariden, Galactooligosacchariden, Sialooligosacchariden, Xylooligosacchariden, Inulin, Gummi arabicum, Guargummi, resistenter Stärke, aus Milch abegeleiteten Oligosacchariden, Pektin und Gemischen davon.

4. Zusammensetzung zur Verwendung nach einem beliebigen der voranstehenden Ansprüche, wobei die Zusammensetzung mindestens 3 Nukleotide umfasst, ausgewählt aus der Gruppe bestehend aus Cytidin-5'-Monophosphat (CMP), Uridin-5'-Monophosphat (UMP), Adenosin-5'-Monophosphat (AMP), Guanosin-5'-Monophosphat (GMP) und Inosin-5'-Monophosphat (IMP).

5. Zusammensetzung zur Verwendung nach einem beliebigen der voranstehenden Ansprüche, wobei die Zusammensetzung zum Fördern von Aufholwachstum in einem kranken Kind dient.

6. Zusammensetzung zur Verwendung nach einem beliebigen der voranstehenden Ansprüche, wobei die Zusammensetzung keine lebenden Bakterien umfasst.

## Revendications

1. Une composition à teneur en énergie élevée comprenant protéine, graisse, des hydrates de carbone assimilables et inassimilables et des nucléotides, laquelle composition présente:
a. une densité d'énergie d'au moins 0,9 kcal/ml et de préférence inférieure à 2,0 kcal/ml; et
b. un composant protéinique fournissant au moins 9,5% du total des calories; et
c. au moins 0,1% en poids d'acides gras polyinsaturés à chaîne longue (LC-PUFAs) exprimé par rapport à la totalité des acides gras, comprenant au moins un LC-PUFA choisi dans le groupe consistant en l'acide eicosapentaénoïque (EPA), l'acide docosahexaenoïque (DHA), l'acide arachidonique (ARA) et l'acide docosapentaenoïque (DPA); et
d. au moins 0,1% en poids d'hydrate de carbone inassimilable, exprimé par rapport au poids à sec de la composition; et
e. au moins 1.10⁻⁴ % en poids de nucléotides, exprimé par rapport au poids à sec de la composition
pour une utilisation dans le traitement et/ou la prévention de problèmes de croissance chez les enfants.

2. La composition pour une utilisation selon la revendication 1, laquelle composition est administrée comme l'est une alimentation par sonde.

3. La composition pour une utilisation selon la revendication 1 ou 2, dans laquelle l'hydrate de carbone inassimilable est choisi dans le groupe consistant en les fructo-oligosaccharides, les galacto-oligosaccharides, les sialo-oligosaccharides, les xylo-oligosaccharides, l'inuline, la gomme arabique, la gomme de guar, l'amidon résistant, les oligosaccharides dérivant du lait, la pectine et leurs mélanges.

4. La composition pour une utilisation selon l'une quelconque des revendications précédentes, laquelle composition comprenant au moins 3 nucléotides choisis dans le groupe consistant en le 5'-monophosphate de cytidine (CMP), le 5'-monophosphate d'uridine (UMP), le 5'-monophosphate d'adénosine (AMP), le 5'-monophosphate de guanosine (GMP) et le 5'-monophosphate d' inosine (IMP).

5. La composition pour une utilisation selon l'une quelconque des revendications précédentes, dans laquelle la composition est pour promouvoir le rattrapage de croissance chez un enfant malade.

6. La composition pour une utilisation selon une quelconque des revendications précédentes, dans laquelle la composition ne comprend pas de bactéries vivantes.
